# EUROPEAN PATENT APPLICATION

(11) **EP 4 703 010 A1**
(43) Date of publication of application: **04.03.2026**
(21) Application number: 24198286.7
(22) Date of filing: 03.09.2024
(51) Int. Cl.: A61N 5/06

(54) **POSITIONING DEVICE FOR POSITIONING A LIGHT-CONDUCTING FIBER IN A CALIBRATION PORT**

(71) Applicant: Omicron-Laserage Laserprodukte GmbH, 63110 Rodgau-Dudenhofen (DE)
(72) Inventor: BAUMANN, Sönke-Nils, 63739 Aschaffenburg (DE)
(74) Representative: Patentanwälte Olbricht Buchhold Keulertz

(57) **Abstract**

The invention relates to a positioning apparatus (100) for positioning a light-guiding fiber (201) in a calibration port (204) of a medical apparatus (201), wherein the positioning apparatus (100) comprises a main body (101) having a base portion (102) and an elongate portion (103), wherein the base portion (102) of the main body (101) has a first end face (104) and wherein the elongate portion (103) of the main body (101) has a second end face (105), and wherein the main body (101) comprises a channel (106) for receiving the light-guiding fiber (201), the channel (106) extending along a longitudinal axis (A) of the main body (101) from the first end face (104) through the base portion (102) and at least partly through the elongate portion (103). The apparatus (100) is characterized in that the base portion (102) of the main body (101) comprises at least one control element (107) for ascertaining the rotational orientation of the light-guiding fiber (201) along the longitudinal axis (A) when the light-guiding fiber (201) is received by the channel (106). Further aspects of the invention relate to a system (200) comprising said apparatus (100), and to a method of calibrating the light source (202) of said system (200).

## Description

The invention relates to a positioning apparatus for positioning a light-guiding fiber in a calibration port of a medical apparatus, to a system with a positioning apparatus, at least one light source, and at least one light-guiding fiber, and to a method for calibrating a light source of said system.

Laser radiation is well-known in medical technology. In addition to the established applications, such as, surgery or ophthalmology, the coherent monochromatic radiation of a laser source is increasingly used in for activation of corresponding drugs or medicaments. A prominent example in this respect is photodynamic therapy (PDT).

As part of PDT, a patient is administered a drug that mainly attaches itself to tumor cells or bacteria. By absorbing electromagnetic radiation of a certain wavelength, the drug is activated, thus causing it to have its desired medical effect on the cells to which it is attached. This is novel way to combat tumors, or to visualize areas of the human body that are affected by certain bacteria.

In order to expose corresponding areas of the human body to electromagnetic radiation or laser radiation, it is known in the prior art to couple corresponding laser radiation into a light-conducting fiber and to couple it out of the light-conducting fiber at the treatment site, such that the target area is exposed to the light. It is crucial, however, that the target area is exposed to a certain radiance (power per area) of the laser, as effective treatment may otherwise not be possible. If, for example, the applied radiance is too low, the drug may not be activated. If the radiance is too high, the treated tissue can be subject to injuries caused by irradiation.

To avoid the above drawbacks, the light power (radiant flux) that is coupled out of a light-conducting fiber is usually calibrated before treatment. For example, the light-conducting fiber may be placed in an integrating sphere, such that the total light power emitted by the fiber is recorded. However, since the fiber will subsequently be used for a medical procedure, sterility of the light-conducting fiber during calibration must be guaranteed. The light-guiding fiber may therefore be surrounded by a sterile sleeve, which serves as a barrier between the sterile fiber and the non-sterile measuring device.

A method for calibrating a light-guiding fiber is known DE 10 2017 112 482 A1. The method comprises a positioning apparatus for positioning the fiber in a calibration port of a medical apparatus. Once the positioning apparatus is inserted into the calibration port and the light-guiding fiber is inserted into the positioning apparatus, radiation coupled out of the fiber emerges through one or more cutouts of the positioning apparatus and is subsequently determined by appropriate sensors of the calibration port, e.g. by photodiodes, photoresistors, phototransistors, and/or photovoltaic light sensors.

However, some fibers are configured to specifically emit light in a specific direction lateral to the fiber. Some fibers may for example emit light in a 90° angle relative to the direction in which the light is guiding through the fiber, i.e. in a direction perpendicular to the light-guiding fiber. In order to detect the light emitted and to calibrate the fiber, it is important that the fiber is correctly aligned in the calibration port of the medical apparatus. Wrong alignment in the calibration port can lead to lower levels of light detection and therefore to erroneous radiance measurements, since not all of the desired amount of light may hit the sensors of the medical apparatus.

The objective technical problem of the present invention is to eliminate the disadvantages of the prior art and to provide an improved positioning apparatus for positioning and aligning a light-guiding fiber in a calibration port.

The problem is solved by a positioning apparatus for positioning a light-guiding fiber in a calibration port of a medical apparatus in accordance with claim 1, by a system comprising said positioning apparatus in accordance with claim 13, and by a method in accordance with claim 16. Special embodiments or beneficial variants of the apparatus are presented in claims 2 to 12. Accordingly, special embodiments or beneficial variants of the system are presented in claims 14 and 15. Special embodiments or beneficial variants of the method are presented in claim 17.

The positioning apparatus comprises a main body having a base portion and an elongate portion, wherein the base portion of the main body has a first end face and wherein the elongate portion of the main body has a second end face, and wherein the main body comprises a channel for receiving the light-guiding fiber, the channel extending along a longitudinal axis of the main body from the first end face through the base portion and at least partly through the elongate portion. The positioning apparatus is characterized in that the base portion of the main body comprises at least one control element for ascertaining the rotational orientation of the light-guiding fiber along the longitudinal axis when the light-guiding fiber is received by the channel.

By ensuring that the light-guiding fiber is inserted into the medical apparatus in its correct rotational orientation, a user of the respective calibration system can ascertain that the light coupled out of the light-guiding fiber at a specific angle does hit the sensors of the medical apparatus, thereby eliminating the risk that only a portion of the desired amount of light emitted from the fiber hits the sensors, or even that no light hits the sensors at all.

According to preferred embodiment, the at least one control element may be a groove in the first end face. This way, the control element is easily visible for the user. Furthermore, a groove allows the user to also track at least part of the light-guiding fiber while it is inserted into the positioning apparatus. Therefore, should the fiber have orientational markings as well, a user may easily make sure that the marking of the fiber and the control element of the positioning apparatus are aligned to each other, thus ensuring that the light coupled out of the fiber at a certain angle hits the sensors as intended. A physical mark, such as a groove, also increases durability of the positioning apparatus, since the groove cannot fade away or otherwise diminish over time.

According to another embodiment, the base portion of the main body may have a greater radius than the elongate portion of the main body. This way, the main body will abut to the medical apparatus when the positioning apparatus is fully inserted, ensuring the right insertion depth of the positioning apparatus within the medical apparatus.

According to another embodiment, at least the elongate portion of the main body may at least partially comprise a semitransparent material and/or is coated with a semitransparent material, such that radiation emitted by the light-guiding fiber can at least partly emerge from the positioning apparatus through the semitransparent material. This way, the positioning apparatus does not require lateral or radial cutouts that would otherwise allow for light emittance, thus minimizing production costs. Instead, the light that is coupled out a specific angle from the light-guiding tube, say at 90°, can permeate the positioning apparatus and hit the sensors at a known power level (depending on the transparency of the material of the elongate portion of the main body). According to this disclosure, "transparent" or "transparency" refers to the physical property of allowing the light coupled out from the light-guiding fiber to pass through the material with only low levels of scattering or absorption of said light. Therefore, in accordance with this disclosure, a "transparent" material is understood to be "not opaque" with respect to the radiation coupled out from the light-guiding fiber. Accordingly, "semitransparent" may be understood to mean that a given material absorbs or scatters some portion of the light passing through (but not all of the light).

When choosing the level of transparency of the material of the positioning apparatus that determines the emission characteristic of the positioning apparatus, care should preferably be taken that some of the laser radiation output coupled from the light-guiding fiber will always be absorbed by the positioning apparatus depending on penetration depth.

According to another embodiment, at least one elongate, lateral cutout may be provided at the elongate portion of the main body. The at least on lateral cutout may facilitate a better ascertainment of the radiance output coupled from the light-guiding fiber, since no radiation will be lost to absorption or scattering. According to another embodiment, the cross section of the lateral cutout may increase from the channel outwards to the side face of the main body. Effectively, this yields a funnel-shaped configuration of the lateral cutout. This funnel-shaped configuration is advantageous in that the generally divergent radiation, which is output coupled from the light-guiding fiber, is not impeded during the propagation thereof by the positioning apparatus or by the opaque material of the positioning apparatus in the region of the channel. Consequently, a funnel-shaped configuration of the cutout improves the accuracy of an ascertainment of the radiance output coupled from the light-guiding fiber.

According to another embodiment, the channel does not penetrate the second end face of the main body. In other words, the channel may end in the elongate portion of the main body. Since the light-guiding fiber may in particular be configured for light emission in a radial direction, e.g. from a specific radial emission point of the fiber, it may be beneficial to assure that the light-guiding fiber can only be inserted into the positioning apparatus at specific depth.

According to another embodiment, the channel may comprise a main section and an end section, wherein the main section has a first diameter D1 that is larger than a second diameter D2 of the end section. In particular, the first diameter D1 may be larger than the maximum diameter of the light-guiding fiber, and the second diameter D2 may be smaller than the maximum diameter of the light-guiding fiber. This way, it is possible to insert a light-guiding fiber that has diameter smaller than D1 and larger than D2 into the channel, such that the light-guiding fiber will abut against the end section of the channel, as it cannot enter the end section. Thus, the light-guiding fiber cannot be involuntarily inserted too deep into positioning apparatus.

Preferably, the first diameter D1 may be just that much larger than the maximum diameter of the fiber, the fiber will fit into the main section of the channel in a snug fit. This ensures that the fiber does not slip out of the channel in the longitudinal direction of the positioning apparatus. A light-guiding fiber with a maximum diameter smaller than D1 and D2, e.g. a light-guiding fiber that emits light at its tip, can be inserted all the way through the end section of the channel until it reaches the second end face of the main body.

According to another embodiment, the main body may have a frontal cutout on the second end face of the elongate portion, wherein the frontal cutout is connected to the end section of the channel. This allows for a radiant flux output coupled out of the fiber in a frontal direction (i.e. from the tip of the light-guiding fiber) to emerge from the positioning apparatus.

According to another embodiment, the diameter of the frontal cutout may increase in the direction of the second end face of the main body. The minimum diameter of the frontal cutout is preferably chosen in such a way that the fiber in the positioning apparatus is only supported at the cladding of the fiber, while the radiation-guiding core of the fiber is completely exposed by the cutout in the longitudinal direction. In any way, the minimum diameter of the frontal cutout should always be smaller than the diameter of the light-guiding fiber at its tip. This prevents fiber from being pushed out of the positioning apparatus through the frontal cutout.

According to another embodiment, an alignment element may be disposed on the second end face of the positioning apparatus. In that case, the frontal cutout may be located within the alignment element. The channel may then extend from the groove in the first end face to the frontal cutout in the alignment element on the second end face. The second diameter D2 may further be substantially halved in the region of the alignment element, resulting in an aperture.

According to another embodiment, the main body may be rotationally symmetric about the longitudinal axis (A) of the main body. This allows for the positioning apparatus to be inserted into the respective port of the medical apparatus with any rotational orientation, thereby providing a degree of freedom and thus facilitating insertion of the positioning apparatus.

According to another embodiment, the main body, at least in one portion, may comprise sterilizable material and/or is coated with a sterilizable material. For example, the channel of the positioning apparatus and the regions of the body of the positioning apparatus adjoining said channel, in particular, are preferably sterilizable. Furthermore, the entire body of the positioning apparatus may consist of a sterilizable material.

According to another embodiment, the sterilizable material may be a plastic, in particular polyoxymethylene. The sterilizable material should also be semitransparent and/or transparent for the light coupled out of the fiber in a radial direction.

Another aspect of the invention relates to a system comprising at least one medical apparatus comprising at least one light source, at least one light-guiding fiber and at least one positioning apparatus as described herein, wherein the light-guiding fiber is connectable to the light source of the medical apparatus in such a way that at least a portion of laser radiation with a defined radiant flux generated by the light source is coupled into the light-guiding fiber, wherein the medical apparatus comprises a calibration port, wherein the calibration port comprises sensor means configured to determine the radiant flux of the laser radiation emerging from the light-guiding fiber, and wherein the at least one calibration port is configured to receive the positioning apparatus.

The light source generates laser radiation having a specific radiant flux and a specific wavelength. By way of example, the light source can be a laser source, for example one or more laser diodes. The laser diodes may be connected to the light-guiding fiber by way of an appropriate optical unit such that the radiant flux or laser radiation emitted by the laser diode is coupled into the light-guiding fiber. By way of example, an optical fiber with a thickness of 200-1200 µm, preferably of 400-600 µm, can be used as a light-guiding fiber. Here, typical radiant fluxes used in the field of PDT lie in the range of 0.25-10 watt, preferably in the range of 1.5-5 watt.

According to another embodiment, the light-guiding fiber may comprise a visual marker for tracing the rotational orientation and/or the depth of the light-guiding fiber when the light-guiding fiber is inserted in the channel of the positioning apparatus. The visual marker facilitates correct alignment of the light-guiding fiber with the control element of the positioning apparatus, thereby assuring correct alignment of the fiber in the medical apparatus, such that the light coupled out from the fiber in a lateral or radial direction can hit the sensor means of the medical apparatus. The visual marker of the light-guiding fiber may for example comprise a first strip printed or otherwise disposed in a longitudinal direction of the fiber and/or second strip in circumferential direction around the fiber.

According to another embodiment, the at least one calibration port may be configured to receive the positioning apparatus in such a way that, by way of the subsequent insertion of the at least one light-guiding fiber into the positioning apparatus, the light-guiding fiber is positioned relative to the sensor means such that the radiant flux of the laser radiation emerging from the light-guiding fiber can be determined by the sensor means.

According to another embodiment, the calibration port may a unit that is separate from the medical apparatus and that is connected to the medical apparatus via one or more data link.

According to another embodiment, the medical apparatus may further comprise an operating element, for example a touch-sensitive display. The operating element allows a user to independently set different parameters of the light be emitted from the light source, such as output power, irradiation time and/or wavelength. A user may further be able to select from preset protocols of output powers, wavelengths and irradiation times, the protocols each simulating different treatment scenarios. The medical apparatus may also be configured to independently determine the corresponding operating parameters of the light source that are necessary for a specific treatment set by the user.

Another aspect of the invention relates to a method for calibrating the light source of a system as presented herein, including the following steps:
(a) connecting the light-guiding fiber to the light source;
(b) inserting the light-guiding fiber into the positioning apparatus;
(c) aligning the visual marker of the light-guiding fiber with the control element of the positioning apparatus;
(d) inserting the positioning apparatus into the calibration port of the medical apparatus;
(e) coupling laser radiation with a defined radiant flux from the light source into the light-guiding fiber;
(f) determining the radiant flux of the laser radiation emerging from the light-guiding fiber within the calibration port with the sensor means of the medical apparatus;
(g) comparing the radiant flux emerging from the light-guiding fiber with the radiant flux coupled into the light-guiding fiber;
(h) if required, adjusting the radiant flux coupled into the light-guiding fiber such that the radiant flux emerging from the light-guiding fiber lies within a defined value range.

Any one of the embodiments, examples or features disclosed herein may be used in combination or separately and in conjunction with any one of the aspects of the disclosed subject matter, mutatis mutandis.

The accompanying drawings illustrate several examples of the subject matter described above. The drawings depict the following:
- Fig. 1: shows an isometric view of a positioning apparatus for positioning a light-guiding fiber in a calibration port of a medical apparatus;
- Fig. 2: shows a lateral view of a positioning apparatus for positioning a light-guiding fiber in a calibration port of a medical apparatus;
- Fig. 3: shows a close-up view of a control element of a positioning apparatus;
- Fig. 4: shows a schematic illustration of a system comprising a medical apparatus and a positioning apparatus;
- Fig. 5: shows a schematic cross-sectional illustration of a system comprising a medical apparatus and a positioning apparatus.

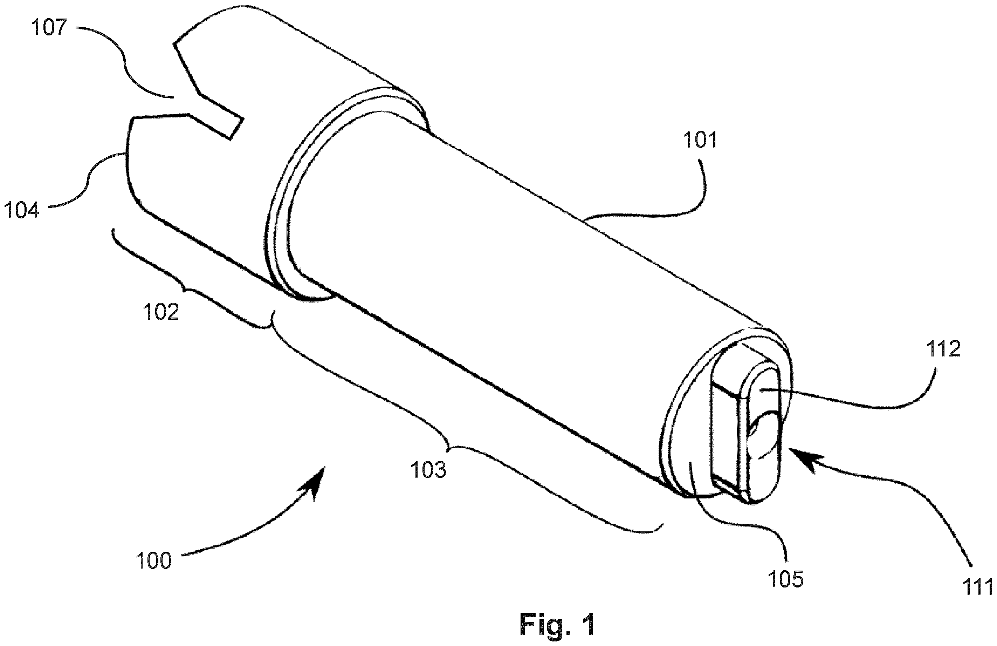

Fig. 1 shows an isometric view of a positioning apparatus 100 for positioning a light-guiding fiber 203 in a calibration port 204 of a medical apparatus 201. The positioning apparatus 100 shown in Fig. 1 has a substantially rotationally symmetric main body 101 that encloses a channel 106 for a light-guiding fiber (not illustrated). The main body 101 has a base portion 102 and an elongate portion 103, which are also both substantially rotationally symmetric.

The base portion 102 of the main body 101 has a first end face 104. Accordingly, the elongate portion 103 of the main body 101 has a second end face 105. The main body 101 comprises a channel 106 which, in case of the embodiment shown in Fig. 1, extends from the first end face 104 through to the second end face 105 along a longitudinal axis A of the main body 101.

The diameters of the base portion 102 and the elongate portion 103 differ from each other. Furthermore, the base portion 102 extends from the first end face 104 over approximately one quarter of the total length of the positioning apparatus 100, while the elongate body 103 extends from a second end face 105 over the remaining length of the positioning apparatus 100 (i.e., approximately three-quarters). The edges of the base portion 102 and the elongate portion 103 of the main body 101 are preferably beveled by a chamfer such that there are no sharp edges on the positioning apparatus 100.

As an exception to the rotationally symmetry, the base portion 102 of the positioning apparatus 100 shown in Fig. 1 comprises a control element 107 in the form of a groove 114 formed into the first end face 104 of the main body 101. The groove 114 extends longitudinally across the first end face 104, essentially cleaving at least the surface of the first end face 104. As shown in Fig. 1, the groove 114 may comprise a funnel-shaped section 115, wherein the width of the funnel decreases in a direction towards the elongate body 103. However, the grove 114 is preferably only funnel-shaped when viewed from a direction along the grove's 114 progression across the surface of the first end face 104. The funnel-shaped section 115 of the groove 114 of Fig. 1 then transitions into a straight section 116, which ends blindly in the base portion 102, with the exception of the channel 106, which continues to protrude through the base portion 102 and the elongate portion 103.

Furthermore, an alignment element 112 is disposed on the second end face 105 of the positioning apparatus 100. In embodiment shown in Fig. 1, the alignment element 112 is an extrusion protruding outward from the second end face 105, said extrusion having rounded corners. The channel 106 passes through the alignment element 112, resulting in the frontal cutout 111 at the tip of the positioning apparatus 100.

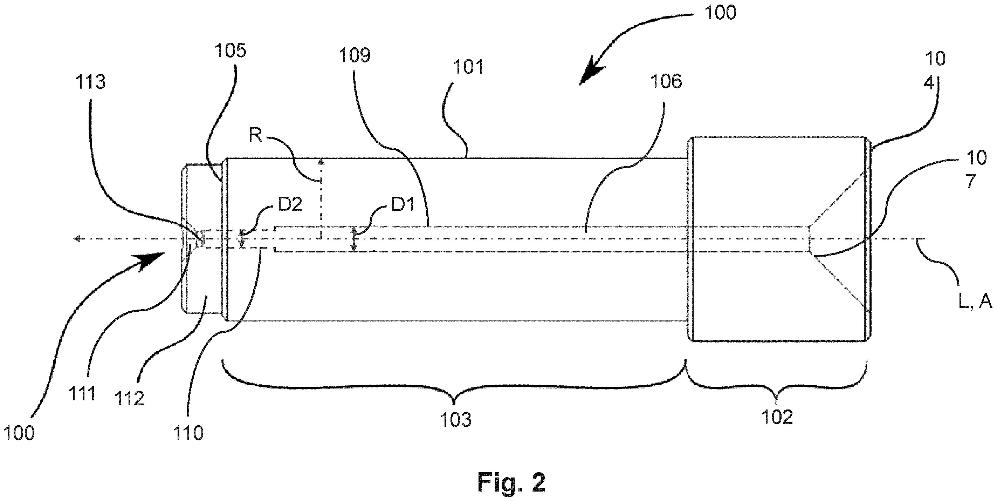

Fig. 2 depicts a lateral view of a positioning apparatus 100, with the grove 114 and the channel 106 being made visible in the illustration through broken lines. The channel 106 extends through the main body 101 along the longitudinal axis A of the positioning apparatus 100. As is shown in Fig. 2, the funnel-shaped section 115 of the grove 114 leads to the channel 104, which then extends through the main body 101 of the positioning apparatus 100 to the second end face 105.

As shown in Fig. 2, the channel 106 may comprise a main section 109 and an end section 110, wherein the main section 109 has a first diameter D1 that is larger than a second diameter D2 of the end section 110. The groove 114 in the first end face 104 of the positioning apparatus 100 transitions into the main section 109 of the channel 106. The main section 109 then progresses through the base portion 102 and through most of the elongate portion 103, before the diameter D1 of the main section 109 abruptly decreases to the diameter D2 of the end section 110. Ideally, the first diameter D2 is just large enough that a light-guided fiber 203 can be inserted in the main section 109 of channel 106 until it reaches the end section 110 of the channel 106 where it cannot enter due to the end section's smaller diameter D2. This prevents the light-guiding fiber 203 from being inserted too deep into positioning apparatus 100 and ensures that the fiber 203 is always inserted into the positioning apparatus 100 with the same depth, provided that its diameter is larger than that of the end section 110. However, a light-guiding fiber 203 having a maximum diameter that is smaller than both D1 and D2, e.g. a light-guiding fiber 203 that emits light at its frontal tip, can be still inserted all the way through the end section 110 of the channel 106 until it reaches an aperture 113.

The channel 106 extends through the whole positioning apparatus 100, i.e. from the groove 114 in the first end face 104 to the frontal cutout 111 in the alignment element 112 on the second end face 105. with the second diameter D2 of the end section 110 of the channel 106 being reduced within the alignment element 112. For example, as shown in Fig. 2, the second diameter D2 is substantially halved in the alignment element 112, resulting in the aperture 113 of the channel 106, which, in terms of its diameter, is preferably adapted to the diameter of the core of a light-guiding fiber 203 inserted into the positioning apparatus 100. Proceeding from this reduced diameter, the channel 106 subsequently widens in the longitudinal direction along the alignment element 112, such that the channel 104 results in the frontal cutout 111.

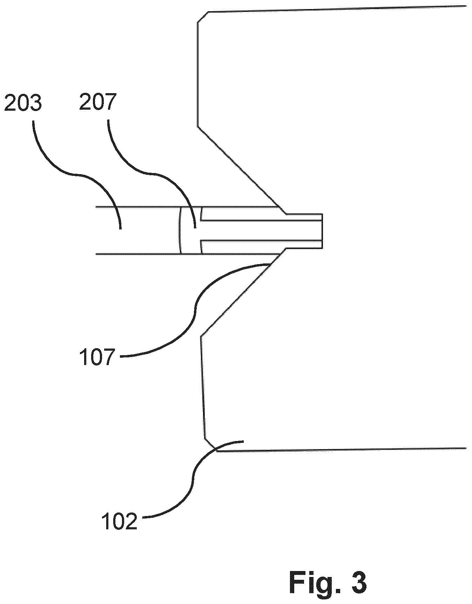

Fig. 3 depicts a close-up view of a control element 107 of a positioning apparatus 100. The control element 107 shown in Fig. 3 is a groove 114 that is disposed in the first end face 104 of the base portion 102. The base portion 102 is depicted in such a way that the groove 114 can be seen along its longitudinal progression across the first end face 104. This way, the funnel-shaped section 115 and the straight section 116 of the groove 114 can be seen.

Furthermore, a light-guiding fiber 203 is shown in Fig. 3, wherein light-guiding fiber 203 is inserted in the positioning apparatus 100. The light-guiding fiber 203 comprises a visual marker 207 for tracing the rotational orientation and the depth of the light-guiding fiber 203 once inserted in the channel 106 of the positioning apparatus 100. The visual marker facilitates 207 correct alignment of the light-guiding fiber 203 with the control element 107 of the positioning apparatus 100, which is a groove 114 in Fig. 3. This way, correct alignment of the fiber 203 in the medical apparatus 201 (not shown) can be ensured, such that the light coupled out from the fiber 203 in a radial direction can hit the sensor means of the medical apparatus 201. As is the case in Fig. 3, the visual marker 207 of the light-guiding fiber 203 comprises a strip in longitudinal direction of the fiber 203 for rotational alignment with the groove 114 and a circumferential strip around the fiber 203 for ascertaining the correct insertion depth of the light-guiding fiber 203 in the channel 106 of the positioning apparatus 100.

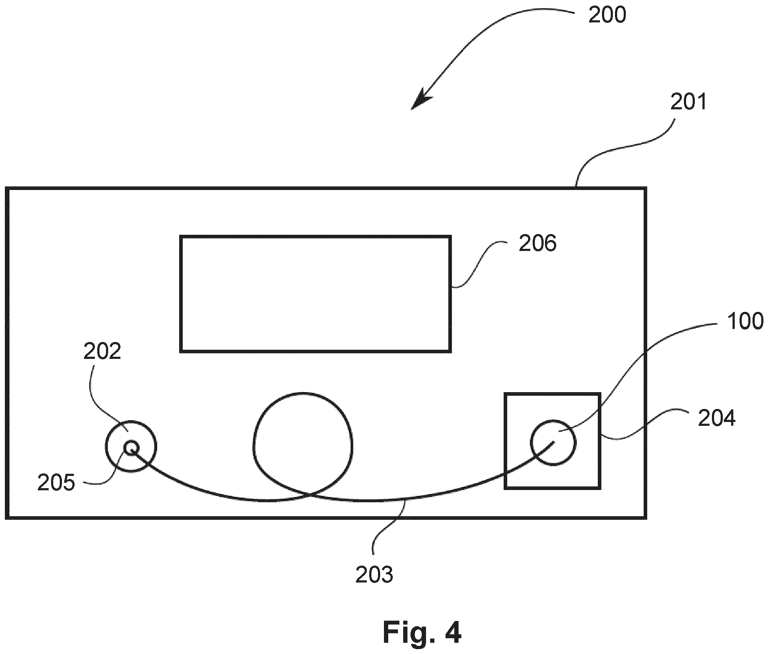

Fig. 4 shows a schematic illustration of a system 200 comprising a medical apparatus 201 and a positioning apparatus 100. The medical apparatus 201 comprises at least one light source 202, at least one light-guiding fiber 203 and at least one positioning apparatus 100 as presented herein. By way of example, the light source 202 can be one or more of a laser diode. The light-guiding fiber 203 is connected to the light source 202 of the medical apparatus 201 in such a way that at least a portion of laser radiation with a defined radiant flux generated by the light source 202 is coupled into the light-guiding fiber 203 and guided through the fiber 203 until it is emitted at a predetermined section of the fiber 203. The medical apparatus 201 further comprises a calibration port 204 configured to receive the positioning apparatus 100. The calibration port 204 also includes light sensor means (not shown) configured to determine the radiant flux of the laser radiation emerging from the light-guiding fiber 203.

The light source 202 is connected to the light-guiding fiber 201 by way of an appropriate optical unit 205, such that the laser radiation emitted by the laser diodes is coupled into the light-guiding fiber 203.

The medical apparatus 201 further comprises an operating element 206, which may for example be a touch-sensitive display. The operating element 206 allows a user to set one or more parameters of the light emitted from the light source 202, such as output power, irradiation time and/or wavelength. Alternatively, a user may also be able to select from preset protocols of output powers, wavelengths and irradiation times, the protocols each simulating different treatment scenarios. The medical apparatus 201 may be configured to independently ascertain the corresponding operating parameters of the light source 202 that are necessary for a specific treatment set by the user.

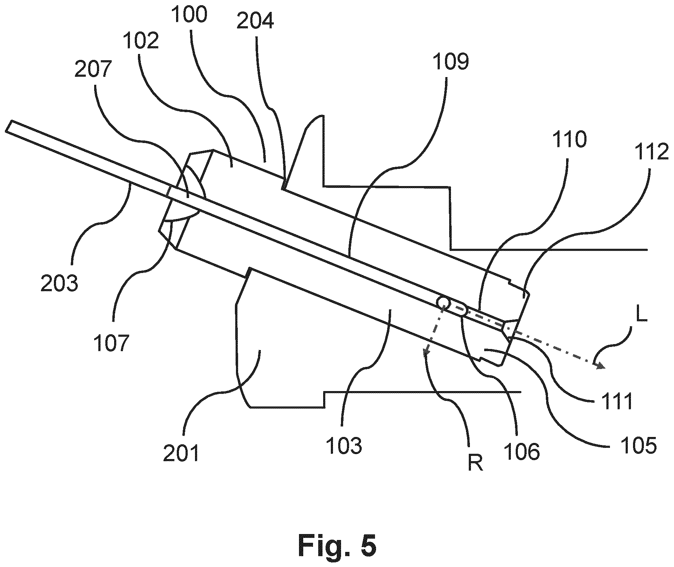

Fig. 5 shows a schematic cross-sectional illustration of a system 200 comprising a medical apparatus 201 and a positioning apparatus 100. In Fig. 5, the light-guiding fiber 203 is already inserted in the positioning apparatus 100 and the positioning apparatus 100 is inserted in the medical apparatus 201. To ensure correct rotational orientation of the fiber 203 in the medical apparatus 201, the fiber bears a visual marker 207 comprising a first strip in a longitudinal direction of the fiber 203 and second strip in circumferential direction around the fiber 203. The control element 107 of the positioning apparatus 100 configured as a groove 114 facilitates correct alignment of the light-guiding fiber 203, since a user can make sure that the visual marker 207 on the fiber 203 is aligned the control element 107. This way, it can be ensured that the light coupled out from the light-guiding fiber 203 at a radial emission point 208 of the fiber 203 will hit the sensor means of the medical apparatus 201.

As is shown in Fig. 5, the light-guiding fiber 203 may be inserted into the channel 106 of positioning apparatus 100 until the fiber 203 abuts against the outer edge of the end section 110 of the channel 106, as the maximum diameter of the light-guiding fiber 203 is bigger than the diameter D2 of the end section 110 of the channel 106.

All features and advantages emerging from the claims, the description and the drawing, including structural details, spatial arrangements and method steps, can be essential to the invention, both on their own and in various combinations.

### Reference numerals

| | | | |
|---|---|---|---|
| 100 | Positioning apparatus | A | Longitudinal axis |
| 101 | Main body | L | Longitudinal direction |
| 102 | Base portion | R | Radial direction |
| 103 | Elongate portion | D1 | First diameter |
| 104 | First end face | D2 | Second diameter |
| 105 | Second end face | | |
| 106 | Channel | | |
| 107 | Control element | | |
| 109 | Main section | | |
| 110 | End section | | |
| 111 | Frontal cutout | | |
| 112 | Alignment element | | |
| 113 | Aperture | | |
| 114 | Groove | | |
| 115 | Funnel-shaped section | | |
| 116 | Straight section | | |
| | | | |
| 200 | System | | |
| 201 | Medical apparatus | | |
| 202 | Light source | | |
| 203 | Light-guiding fiber | | |
| 204 | Calibration port | | |
| 205 | Optical unit | | |
| 206 | Operating element | | |
| 207 | Visual marker | | |
| 208 | Radial emission point | | |

## Claims

1. A positioning apparatus (100) for positioning a light-guiding fiber (201) in a calibration port (204) of a medical apparatus (201), wherein the positioning apparatus (100) comprises a main body (101) having a base portion (102) and an elongate portion (103), wherein the base portion (102) of the main body (101) has a first end face (104) and wherein the elongate portion (103) of the main body (101) has a second end face (105), and wherein the main body (101) comprises a channel (106) for receiving the light-guiding fiber (201), the channel (106) extending along a longitudinal axis (A) of the main body (101) from the first end face (104) through the base portion (102) and at least partly through the elongate portion (103), **characterized in that** the base portion (102) of the main body (101) comprises at least one control element (107) for ascertaining the rotational orientation of the light-guiding fiber (201) along the longitudinal axis (A) when the light-guiding fiber (201) is received by the channel (106).

2. The positioning apparatus (100) according to claim 1, **characterized in that** at least one control element (107) is a groove (114) in the first end face (104).

3. The positioning apparatus (100) according to any of the preceding claims, **characterized in that** the base portion (102) of the main body (101) has a greater radius than the elongate portion (103) of the main body (101).

4. The positioning apparatus (100) according to any of the preceding claims, **characterized in that** the positioning apparatus (100) comprises at least one light source (108) for the light-guiding fiber (201).

5. The positioning apparatus (100) according to any of the preceding claims, **characterized in that** at least the elongate portion (103) of the main body (101) at least partially comprises a semitransparent material and/or is coated with a semitransparent material, such that radiation emitted by the light-guiding fiber (201) can at least partly emerge from the positioning apparatus (100) through the semitransparent material.

6. The positioning apparatus (100) according to any of the preceding claims, **characterized in that** the channel (106) does not penetrate the second end face (105) of the main body (101).

7. The positioning apparatus (100) according to any of the preceding claims, **characterized in that** the channel (106) comprises a main section (109) and an end section (110), wherein the main section (109) has a first diameter (D1) that is larger than a second diameter (D2) of end section (110).

8. The positioning apparatus (100) according to claim 7, **characterized in that** the main body (101) has a frontal cutout (111) on the second end face (105) of the elongate portion (103), wherein the frontal cutout (111) is connected to the end section (110) of the channel (106).

9. The positioning apparatus (100) according to claim 8, **characterized in that** the diameter of the frontal cutout (111) increases in the direction of the second end face (105) of the main body (101).

10. The positioning apparatus (100) according to any of the preceding claims, **characterized in that** the main body (101) is rotationally symmetric about the longitudinal axis (A) of the main body (101).

11. The positioning apparatus (100) according to any of the preceding claims, **characterized in that** the main body (101), at least in one portion, comprises sterilizable material and/or is coated with a sterilizable material.

12. The positioning apparatus (100) according to claim 11, **characterized in that** the sterilizable material is a plastic, in particular polyoxymethylene.

13. A system (200) comprising at least one medical apparatus (201) comprising at least one light source (202), at least one light-guiding fiber (203) and at least one positioning apparatus (100) as claimed in any one of the preceding claims, wherein the light-guiding fiber (203) is connectable to the light source (202) of the medical apparatus (200) in such a way that at least a portion of laser radiation with a defined radiant flux generated by the light source (202) is coupled into the light-guiding fiber (203), wherein the medical apparatus (201) comprises a calibration port (204), wherein the calibration port (204) comprises sensor means configured to determine the radiant flux of the laser radiation emerging from the light-guiding fiber (203), wherein the at least one calibration port (204) is configured to receive the positioning apparatus (100).

14. The system (200) according to claim 13, **characterized in that** the light-guiding fiber (203) comprises a visual marker (207) for tracing the rotational orientation of the light-guiding fiber (201) when the light-guiding fiber (201) is inserted in the channel (106) of the positioning apparatus (100).

15. The system (200) according to any of claims 13 or 14, **characterized in that** the at least one calibration port (204) is configured to receive the positioning apparatus (100) in such a way that, by way of the subsequent insertion of the at least one light-guiding fiber (203) into the positioning apparatus (100), the light-guiding fiber (203) is positioned relative to the sensor means such that the radiant flux of the laser radiation emerging from the light-guiding fiber (203) can be determined by the sensor means.

16. A method for calibrating the light source (202) of a system (200) as claimed in any one of claims 13 to 15, including the following steps:
(a) connecting the light-guiding fiber (203) to the light source (202);
(b) inserting the positioning apparatus (100) into the calibration port (204) of the medical apparatus (200);
(c) inserting the light-guiding fiber (203) into the positioning apparatus (100);
(d) aligning the visual marker of the light-guiding fiber (203) with the control element (107) of the positioning apparatus (100);
(e) coupling laser radiation with a defined radiant flux from the light source (202) into the light-guiding fiber (203);
(f) determining the radiant flux of the laser radiation emerging from the light-guiding fiber (203) within the calibration port (204) with the sensor means of the medical apparatus (201);
(g) comparing the radiant flux emerging from the light-guiding fiber (203) with the radiant flux coupled into the light-guiding fiber (203).

17. The method of claim 17, further comprising the following step:
(h) adjusting the radiant flux coupled into the light-guiding fiber (203) from the light source (202) when the radiant flux emerging from the light-guiding fiber (203) lies outside a defined value range, such that the radiant flux emerging from the light-guiding fiber (203) lies within said value range after adjustment.
